Europäisches Patentamt

European Patent Office

Office européen des brevets

(19)

(11) Veröffentlichungsnummer: **0 199 091 B1**

(12) **EUROPÄISCHE PATENTSCHRIFT**

(45) Veröffentlichungstag der Patentschrift:
31.01.90

(21) Anmeldenummer: 86103754.7

(22) Anmeldetag: 19.03.86

(51) Int. Cl.⁴: **C 12 N 15/00**, C 12 P 21/00,
C 07 K 15/00, A 61 K 39/245,
G 01 N 33/53 // (C12R1/19, 1:07,
1:38, 1:465, 1:04, 1:85, 1:78)

(54) Verfahren zur Herstellung eines für Herpex simplex Virus Typ 2 spezifischen Antigens, dazu geeignetes Mittel und Verwendung dieses Antigens.

(30) Priorität: 25.03.85 DE 3510734

(43) Veröffentlichungstag der Anmeldung:
29.10.86 Patentblatt 86/44

(45) Bekanntmachung des Hinweises auf die Patenterteilung:
31.01.90 Patentblatt 90/5

(84) Benannte Vertragsstaaten:
AT BE CH DE FR GB IT LI LU NL SE

(56) Entgegenhaltungen:
EP-A- 0 100 521
EP-A- 0 101 655

(73) Patentinhaber: BEHRINGWERKE Aktiengesellschaft,
Postfach 1140, D-3550 Marburg 1 (DE)

(72) Erfinder: Bröker, Michael, Dr., Lindenweg 16,
D-3550 Marburg (DE)
Erfinder: Amann, Egon, Dr., Sachsenring 8,
D-3550 Marburg (DE)

(74) Vertreter: Klein, Otto, Dr. et al, Hoechst AG Zentrale
Patentabteilung Postfach 80 03 20, D-6230 Frankfurt am
Main 80 (DE)

## Beschreibung

Die Erfindung betrifft eine rekombinante Desoxyribonucleinsäure, die den genetischen Code für ein antigenes Protein aus Herpes simplex Virus Typ 2 (HSV-2) enthält, eine Zelle, die jene rekombinante DNA enthält und dieses antigene Protein oder Teile desselben produziert, die Verwendung jener rekobinanten DNA oder dieser Zelle zur Herstellung dieses Proteins sowie Verfahren zur Herstellung des Proteins und die Verwendung dieses antigenen Proteins.

Ein Protein, das die Antikörperbildung gegen HSV-2 zu induzieren vermag, ist von großem Interesse für die Herstellung von Impfstoffen zur Prophylaxe und Therapie von Erkrankungen, die durch HSV-2 verursacht werden. Untersuchungen in den letzten Jahren lassen vermuten, daß bestimmte Tumoren durch Herpesviren (HV) hervorgerufen werden können.

Deshalb ist an einen Impfstoff gegen HSV-2 die Forderung zu stellen, daß dieser frei sein muß von biologisch aktiver viraler DNA.

Die konventionelle Herstellung eines diesen Forderungen entsprechenden Impfstoffes würde aufwendige Reinigungsschritte verlangen, die durch Synthese antigener Komponenten von HSV-2 zu umgehen sind. Als antigene Komponenten von HSV wurden bisher einige Glykoproteine aus der Hülle des Virus beschrieben, deren Genorte auf der HSV-DNA zugeordnet und deren DNA-Sequenz ermittelt werden konnten, beispielsweise gD von HSV-1 (gD-1) und von HSV-2 (gD-2). Gentechnologisch hergestelltes gD-1 kann in Mäusen HV-spezifische Antikörper induzieren und Schutz gegen HSV-1 und HSV-2 vermitteln.

Im Gegensatz zu gD, das vorwiegend typengemeinsame antigene Determinanten trägt, sind gC von HSV-1 (gC-1) und HSV-2 (gC-2) als Gycoproteine mit vorwiegend typenspezifischen antigenen Determinanten beschrieben. Die Genorte und DNA-Sequenzen von gC-1 und gC-2 sind ebenfalls bekannt. Antigene Determinanten von gC-1 konnten in E. coli synthetisiert werden. Das Verfahren zur Herstellung und die Verwendung dieses Antigens ist in EP 0 100 521 A2 beschrieben.

In Analogie zu gC-1 kann vermutet werden, daß gC-2 von Bedeutung für die Bildung protektiver Antikörper gegen HSV-2-Infektionen ist.

Es wurde gefunden, daß gC-2 dadurch hergestellt werden kann, daß der gC-2 codierende Genabschnitt aus HSV-2 isoliert und in einen Mikroorganismus transferiert wird, in dem diese DNA repliziert und exprimiert wird. Im folgenden wird ein Verfahren zur Expression von gC-2-spezifischen antigenen Determinanten beschrieben, die aber nicht nur als Impfstoff gegen HSV-2 dienen, sondern auch in der Diagnostik Anwendung finden können.

Gegenstand der Erfindung ist deshalb ein Verfahren zur Herstellung eines Polypeptides mit einer antigenen Determinante des Herpes simplex Virus, indem man aus der Desoxyribonucleinsäure dieses Virus einen Genomabschnitt isoliert, der für ein solches Polypeptid codiert, den Genabschnitt enzymatisch an die Desoxyribonucleinsäure eines Vektors bindet, diese kombinierte Desoxyribonucleinsäure in eine Zelle einbringt, in welcher die kombinierte Desoxyribonucleinsäure die Produktion dieses Polypeptids bewirkt, und dieses Polypeptid gewinnt, dadurch gekennzeichnet, daß der Genomabschnitt das Sall-Fragment (Genomabschnitt etwa 0,62 - 0,64 map units) von Herpes simplex Virus Typ 2 ist.

Vorzugsweise ist der Genomabschnitt ein Teil des Sall-Fragmentes der HSV-2 DNA, welches für gC codiert.

Der Vektor ist vorzugsweise Plasmid pBR322, ein Plasmid der pUC-Serie oder ein Derivat derselben.

Vorteilhafterweise kann der Vektor ein SV40-Vektor oder ein boviner Papillomavirus-Vektor sein.

Die kombinierte Desoxyribonucleinsäure kann vorteilhafterweise die gesamte für gC codierende Sequenz oder Teile derselben und lacZ oder Teile von lacZ enthalten, so daß ein Fusionsprotein aus gC-2 und lacZ erhalten wird.

Die zur Expression verwendete Zelle kann eine tierische oder humane Zelle sein oder ein Mikroorganismus.

Vorzugsweise sind es Zellen von Escherichia coli. Es können auch Zellen eines Mikroorganismus der Gattung Bacillus, Pseudomonas, Streptomyces oder Actinomyces oder einer Hefe der Gattung Saccharomyces, Kluyveromyces, Schizosaccharomyces oder Hansenula sein.

Im folgenden wird das erfindungsgemäße Verfahren näher beschrieben.

### 1. *Gewinnung virus-spezifischer Nucleinsäure und Klonierung des gC-2-Gens*

Die virologischen Methoden (Vermehrung und Isolierung des Virus) und molekularbiologischen Methoden (Klonierung von DNA-Fragmenten in Plasmide) sind allgemein bekannt und in Handbüchern beschrieben (Maniatis et al., 1982, Molecular Cloning, A Laboratory Mannual, Cold Spring Harbor Laboratory, Cold Spring Harbor, N.Y.) und wurden in EP 0 100 521 A2 auf die Klonierung des gC-1-Gens angewendet.

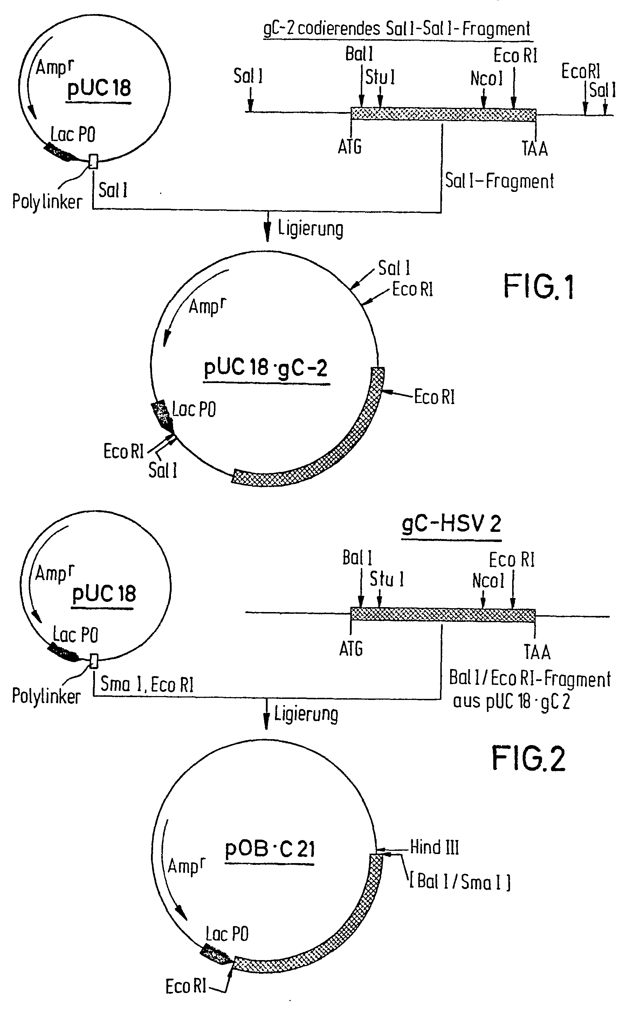

Das gC-2 codierende Sall-DNA-Fragment von HSV-2 wurde in pUC 18 (Vieira and Messing, 1982, Gene, 19, 259-268) kloniert (Fig. 1, pUC 18.gC-2).

### 2. *Konierung von gC-2-codierenden Anteilen in pUC 18*

Das etwa 1200 bp große Ball/EcoRl-Fragment von pUC18.gC-2, das etwa 82% des gC-2 codiert, wurde nach Spaltung des Plasmids mit SmaI und EcoRl in pUC 18 kloniert (Fig. 2). Das neu entstandene Plasmid pOB.C21 enthält den gC-2-codierenden Genabschnitt (Aminosäuren 25-418) ohne die aminoterminale Leadersequenz und die carboxyterminale Membranverankerungssequenz. Das Fehlen dieser hydrophoben Proteinanteile sollte die Expression des gC-2 in Bakterien erleichtern, da solche Sequenzen erfahrungsgemäß das Wachstum von Bakterien negativ beeinflussen können. Das gC-2-codierende Ball/Eco-Fragment ist im pOB.C21 von verschiedenen Erkennungssequenzen für Endonucleasen flankiert und kann mit geeigneten Enzymen aus dem Plasmid herausgespalten und in andere Plasmide so eingesetzt werden, daß es die Synthese des entsprechenden Proteins bewirken kann.

### 3. Expression von gC-2

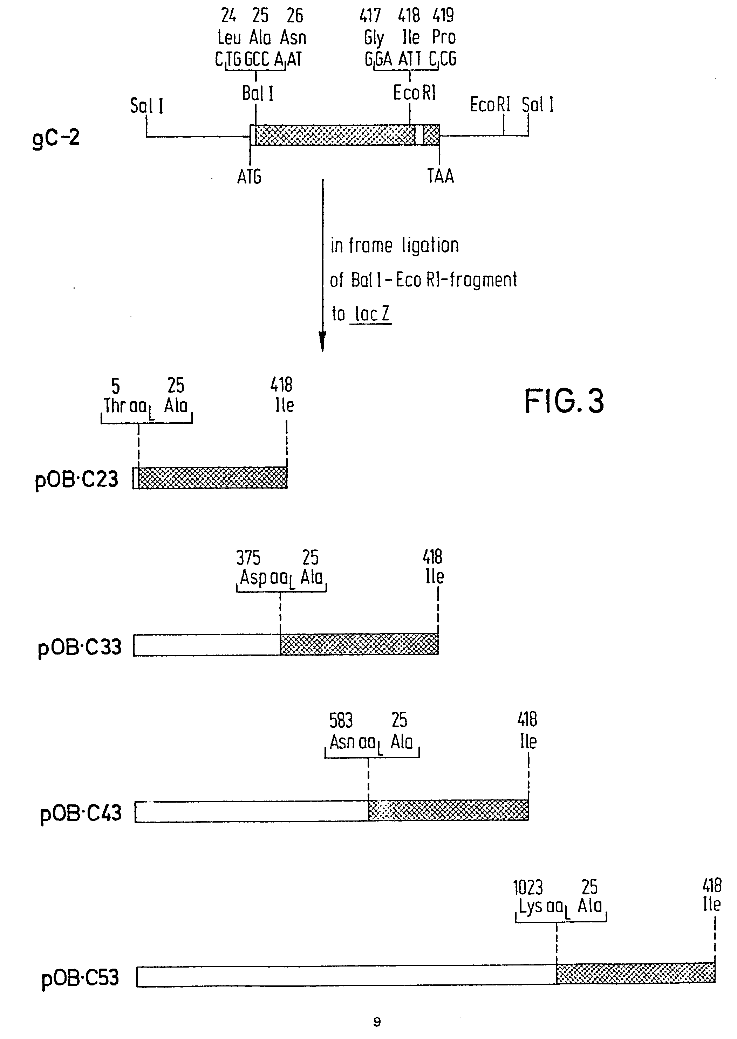

Im folgenden wird die Herstellung von Proteinen beschrieben, die aus gC-2 und einem verschieden großen Anteil von lacZ (Kalnins et al., 1983, EMBO J. 4, 593-597) bestehen (Fig. 3). pOB.C23 wurde erhalten, indem das gC-2-codierende BamHI/EcoRI-Fragment aus pOB.C21 in pUC 19, das mit BamHI und EcoRI abgebaut worden war, ligiert wurde.

pOB.C23 codiert für ein β-gal: :gC-2-Fusionsprotein mit einem β-gal-Anteil von nur 4 Amiosäuren. Um pOB.C33 zu erhalten, wurde das gleiche gC-2-codierende BamHI/EcoRI-Fragment aus pOB.C21 in pBD-2 cloniert, das mit BamHI und HindIII gespalten worden war. Die EcoRI- und die HindIII-Stelle waren vor der Ligierung mit Polymerase I (large fragment) und dNTP «aufgefüllt» worden. Der Anteil an β-gal, der durch pBD2 codiert wird, beträgt 375 Aminosäuren. pBD2 war entstanden durch Ligierung des den Aminoterminus codierenden lacZ-Anteils auf dem EcoRI/EcoRV-Fragment von pUK 230 (Koenen et al., 1982, EMBO J., 1, 509-512) in pUC8 nach Hydrolyse des Plasmids mit SmaI und EcoRI. pOB.C33 codiert somit für das Fusionsprotein β-gal: :gC-2, dessen β-gal-Anteil aus 375 Aminosäuren besteht. pOB.C43, das einen β-gal-Anteil von 583 Aminosäuren des β-gal: :gC-2 Fusionsproteins codiert, wurde wie folgt hergestellt: pWR 590 (Guo et al., 1984, Gene, 29, 251 - 254) wurde mit SalI verdaut, mit Polymerase I und mit BamHI behandelt. In dieses Plasmid wurde nun das gC-2-codierende Fragment von pOB.C21 nach Hydrolyse mit EcoRI, Polymerase I-Behandlung und BamHI-Hydrolyse eingesetzt.

Um pOB.C53 zu erhalten, wurde das gC-2-codierende DNA-Fragment ebenso modifiziert wie bei der Bildung von pOB.C43 und in pUR 278 (Rüther und Müller-Hill, 1983, EMBO J., 2, 1791 - 1794) kloniert, das einer Vorbehandlung mit XbaI, Polymerase I und BamHI unterzogen worden war. Somit codiert pOB.C53 für ein Protein, das aus der gesamten β-gal und gC-2 besteht.

E. coli-Zellen, die die Plasmide pOB.C23, pOB.C33, pOB.C43 oder pOB.C53 enthalten, wurden mit $^{35}$S-Methionin markiert und die synthetisierten Produkte identifiziert. Bis auf pOB.C23 sind alle Plasmide in der Lage, in E. coli die Synthese eines vom lac-Promoter kontrollierten Fusionsproteins vom Typ β-gal: :gC-2 zu bewirken, das von anti-HSV-2-Ziegen-Antiserum, nicht aber von anti-HSV-1-Ziegen-Antiserum erkannt wird. gC-2 wird also in E. coli durch Fusion mit verschieden großen β-gal-Anteilen vor Proteolyse geschützt und ist immunoreaktiv.

Weitere Möglichkeiten, gC-2 stabil in E. coli zu exprimieren, bestehen darin, ein Fusionsprotein, bestehend aus gC-2 und βgal zu synthetisieren, in dem der gC-2-Anteil nicht am Carboxyterminus, sondern am Aminoterminus von βgal gebunden ist (gC2: :βgal) oder in dem gC2 von βgal beidseitig flankiert ist (βgal: :gC-2: :βgal). Nicht nur βgal, sondern auch andere Proteine, wie z.B. das Bakteriophagen-Repressorprotein cI oder Teile desselben können als Bestandteil von Fusionsproteinen heterologe Proteine in E. coli vor Proteolyse schützen (cI: :gC-2). Als sehr stabil haben sich dreiteilige Fusionsproteine vom Typ cI: :Gen X: :βgal erwiesen, so daß z.B. auch eine Synthese von gC-2 als Fusionsprotein vom Typ cI: :gC-2: :βgal als geeignet erscheint.

Die β-gal enthaltenden Fusionsproteine können über Affinitäts-Chromatographie oder Immunabsorptionssäulen gereinigt werden und können, wenn der β-gal-Anteil nicht erwünscht ist, einer Bromcyanspaltung oder proteolytischen Spaltung unterworfen werden, so daß gC-2-Peptide isoliert werden können, die frei von anderen Bestandteilen sind. Gegenüber der Gesamt-βgal verkürzte β-gal-Anteile als Bestandteil von Fusionsproteinen haben den Vorteil, daß bei den Reinigungsprozessen nach der Bromcyanspaltung weniger Peptide entstehen und das gC-2-spezifische Peptid einfacher zu reinigen ist.

Gezielte proteolytische Spaltung zur Abtrennung von unerwünschten Antigenanteilen der gC-2-Fusionsproteine läßt sich auch durch den Einbau von Oligonukleotiden, die die Erkennungssequenzen spezifischer Proteasen enthalten, in die Expressionsplasmide erzielen. Solche spezifischen Erkennungsregionen können sein:

Ile-Glu-Gly-Arg u.a. für Faktor XIIIa; Phe-Val-Arg oder Gly-Pro-Arg u.a. für Thrombin, Val-Gly-Arg oder Glu-Gly-Arg u.a. für Urokinase oder Lys-Gly-Arg für CI-Esterase. Nach der Behandlung der gC-2-Fusionsproteine mit der jeweiligen Protease lassen sich dann affinitätschromatographisch aus dem entstandenen Peptid-Gemisch die gewünschten gC-2-Antigene in reiner Form gewinnen.

Zur Herstellung immunrelevanter gC-2-Antigene können auch eukaryotische Zellen (Hefen, animale oder humane Zellinien) verwendet werden. Dazu wird das gC-2-Gen dahingehend verändert, daß die hydrophoben Aminosäuresequenzen am COOH-Terminus, die zur Verankerung des Proteins in der Zellmembran (oder Virushülle) dienen, entfernt werden. Dies geschieht durch Abspalten der entsprechenden codierenden DNA-Abschnitte des gC-2-Gens durch Restriktionsendonukleasen und durch Einführen eines synthetischen Stop-Codons (TAG, TGA, TAA). Das so veränderte Gen wird nun in einen Expressionsvektor, beispielsweise SV40-Vektoren oder Bovine Papilloma-Virus (BPV)-Vektoren unter der Kontrolle von effizienten, zum Teil regulierbaren Promotoren (zum Beispiel Metallothionein-Promoter) integriert. Das entstandene rekombinante Plasmid wird nun mittels Transfektion in geeignete Wirtszellen eingebracht. Dazu verwendet man in der Regel ein selektierbares Markergen, zum Beispiel Dihydrofolatreduktase im Falle von dhfr[-]-Zellen (zum Beispiel CHO-Zellen) und anschließende Amplifizierung mit Methotrexat, HSV-1, Thymidinkinase im Falle von tk[-]-Zellen (zum Beispiel Mausfibroblasten-Zellen, «L-Zellen»), Antibiotikaresistenzgene, zum Beispiel gegen G418, oder auch transformierende Gene (Onkogene), zum Beispiel im Falle der Focusbildung nach BPV-Transfektion. Das Markergen kann mit dem gC-2-Gen zusammen auf einem Plasmid lokalisiert, aber auch auf einem zweiten Plasmid codiert sein und dann durch Kotransfektion in die Wirtszelle gebracht werden. Auf diese Weise können Zellinien etabliert werden, die ein um die Ankersequenz verkürztes gC-2-Antigen ausscheiden, welches sich dann aus den Kulturüberständen gewinnen läßt. Die Expression von gC-2 in animalen Zellen hat den Vorteil des korrekten Prozessierens, zum Beispiel Glykosylierung und damit einer möglicherweise verbesserten Antigenität, etwa durch erhöhte Stabilität in der Zirkulation nach einer Impfung.

Wegen seiner antigenen Eigenschaften ist ein solches Protein geeignet zur Gewinnung von Antiseren und/oder Impfstoffen, gegebenenfalls unter Verwendung von Adjuvantien und üblichen Hilfsstoffen. Das hergestellte rekombinante gC-2 kann allein als HSV-2-spezifischer Impfstoff dienen oder anderen Glycoproteinen, wie beispielsweise gB oder gD beigemischt werden, so daß der Impfstoff sowohl gegen HSV-1 als auch HSV-2 wirkt. Es kann auch für diagnostische Zwecke verwendet werden, beispielsweise zum Nachweis von Antikörpern gegen HSV-2. Antiseren gegen ein solches Protein können zum Nachweis von HSV-2 selbst dienen.

## Patentansprüche

1. Verfahren zur Herstellung eines Polypeptides mit antigenen Determinanten des Herpes simplex Virus, indem man aus der Desoxyribonucleinsäure dieses Virus einen Genomabschnitt isoliert, der für ein solches Polypeptid codiert, den Genabschnitt enzymatisch an die Desoxyribonucleinsäure eines Vektors bindet, diese kombinierte Desoxyribonucleinsäure in eine Zelle einbringt, in welcher die kombinierte Desoxyribonucleinsäure die Produktion dieses Polypeptids bewirkt, und dieses Polypeptid gewinnt, dadurch gekennzeichnet, daß der Genomabschnitt das Sall-Fragment (Genomabschnitt etwa 0,62 - 0,64 map units) von Herpes simplex Virus Typ 2 ist.

2. Verfahren nach Anspruch 1, dadurch gekennzeichnet, daß der Genomabschnitt ein Teil des Sall-Fragmentes (Genomabschnitt etwa 0,62 - 0,64 map units) der HSV-2 DNA ist, welcher für gC codiert.

3. Verfahren nach Anspruch 1, dadurch gekennzeichnet, daß der Vektor das Plasmid pBR322, ein pUC oder ein Derivat derselben ist.

4. Verfahren nach Anspruch 1, dadurch gekennzeichnet, daß die kombinierte Desoxyribonucleinsäure aus dem HSV-2-Genomabschnitt von etwa 0,62 - 0,64 map units und einem Teil der Vektoren pBR322 oder ein pUC besteht.

5. Verfahren nach Anspruch 1, dadurch gekennzeichnet, daß die kombinierte Desoxyribonucleinsäure die gesamte für gC codierende Sequenz oder Teile derselben und lacZ oder Teile von lacZ enthält.

6. Verfahren nach Anspruch 1, dadurch gekennzeichnet, daß die Zelle Escherichia coli ist.

7. Verfahren nach Anspruch 1, dadurch gekennzeichnet, daß die Zelle ein Mikroorganismus der Gattung Bacillus, Pseudomonas, Streptomyces oder Actinomyces ist, oder eine Hefe der Gattung Saccharomyces, Kluyveromyces, Schizosaccharomyces oder Hansenula.

8. Verfahren nach Anspruch 1, dadurch gekennzeichnet, daß die Zelle eine tierische oder humane Zelle ist.

9. Verfahren nach Anspruch 1, dadurch gekennzeichnet, daß der Vektor ein SV40-Vektor oder ein boviner Papillomavirus-Vektor ist.

10. Polypeptid erhältlich nach Anspruch 1.

11. Verwendung eines Polypeptides nach Anspruch 1 zur Gewinnung eines Antigenserums und/oder zur Herstellung eines Impfstoffes oder eines diagnostischen Mittels.

## Claims

1. A process for the preparation of a polypeptide having antigenic determinants of the herpes simplex virus, by isolation of a section of the genome, which codes for this type of polypeptide, from the deoxyribonucleic acid of this virus, binding of the section of the genome enzymatically to the deoxyribonucleic acid of a vector, introduction of this combined deoxyribonucleic acid into a cell in which the combined deoxyribonucleic acid brings about the production of this polypeptide, and obtaining of this polypeptide, which process comprises the section of the genome being the Sall fragment (section of about 0.62 - 0.64 map units of the genome) of herpes simplex virus type 2.

2. The process as claimed in claim 1, wherein the section of the genome is a part of the Sall fragment (section of about 0.62 - 0.64 map units of the genome) of the HSV-2 DNA which codes for gC.

3. The process as claimed in claim 1, wherein the vector is the plasmid pBR322, a pUC or a derivative thereof.

4. The process as claimed in claim 1, wherein the combined deoxyribonucleic acid is composed of the section of about 0.62 - 0.64 map units of the HSV-2 genome and of a part of the vectors pBR322 or pUC.

5. The process as claimed in claim 1, wherein the combined deoxyribonucleic acid contains the entire sequence coding for gC, or parts thereof, and lacZ or parts of lacZ.

6. The process as claimed in claim 1, wherein the cell is Escherichia coli.

7. The process as claimed in claim 1, wherein the cell is a microorganism of the genus Bacillus, Pseudomonas, Streptomyces or Actinomyces, or a yeast of the genus Saccharomyces, Kluyveromyces, Schizosaccharomyces or Hansenula.

8. The process as claimed in claim 1, wherein the cell is an animal or human cell.

9. The process as claimed in claim 1, wherein the vector is an SV40 vector or a bovine papilloma virus vector.

10. A polypeptide obtainable as claimed in claim 1.

11. The use of a polypeptide as claimed in claim 1 for obtaining an antigenic serum and/or for the preparation of a vaccine or of a diagnostic agent.

## Revendications

1. Procédé pour la préparation d'un polypeptide comportant des déterminants antigéniques de l'herpès simplex virus, dans lequel on isole à partir de l'acide désoxyribonucléique de ce virus un fragment génomique qui code pour un tel polypeptide, on fixe par voie enzymatique le fragment génomique sur l'acide désoxyribonucléique d'un vecteur, on introduit cet acide désoxyribonucléique combiné dans une cellule dans laquelle l'acide désoxyribonucléique combiné provoque la production de ce polypeptide, puis on recueille ce polypeptide, caractérisé en ce que le fragment génomique est le fragment Sall [fragment génomique d'environ 0,62 - 0,64 map unit (unité cartographique)] de l'herpès simplex virus de type 2.

2. Procédé selon la revendication 1, caractérisé en ce que le fragment génomique est une partie du fragment Sall (fragment génomique d'environ 0,62 - 0,64 map unit) de l'ADN de HSV-2, qui code pour gC.

3. Procédé selon la revendication 1, caractérisé en ce que le vecteur est le plasmide pBR322, un pUC ou un dérivé de ceux-ci.

4. Procédé selon la revendication 1, caractérisé en ce que l'acide désoxyribonucléique combiné est constitué du fragment génomique de HSV-2 d'environ 0,62 - 0,64 map unit et d'une partie des vecteurs pBR322, ou d'un pUC.

5. Procédé selon la revendication 1, caractérisé en ce que l'acide désoxyribonucléique combiné contient la séquence totale codant pour gC ou des parties de celle-ci et lacZ ou des parties de lacZ.

6. Procédé selon la revendication 1, caractérisé en ce que la cellule est Escherichia coli.

7. Procédé selon la revendication 1, caractérisé en ce que la cellule est un micro-organisme appartenant au genre Bacillus, Pseudomonas, Streptomyces ou Actinomyces ou une levure appartenant au genre Saccharomyces, Kluyveromyces, Schizosaccharomyces ou Hansenula.

8. Procédé selon la revendication 1, caractérisé en ce que la cellule est une cellule animale ou humaine.

9. Procédé selon la revendication 1, caractérisé en ce que le vecteur est un vecteur SV40 ou un vecteur de type papillomavirus bovin.

10. Polypeptide préparable selon la revendication 1.

11. Utilisation d'un polypeptide selon la revendication 1, pour l'obtention d'un sérum antigénique et/ou pour la préparation d'un vaccin ou d'un agent de diagnostic.

FIG.1

FIG.2

FIG.3